(19) Europäisches Patentamt
European Patent Office
Office européen des brevets

(11) EP 4 501 978 A1

# (12) EUROPEAN PATENT APPLICATION
published in accordance with Art. 153(4) EPC

(43) Date of publication:
**05.02.2025 Bulletin 2025/06**

(21) Application number: **23778998.7**

(22) Date of filing: **17.02.2023**

(51) International Patent Classification (IPC):
***C08F 20/38*** (2006.01) ***B32B 17/10*** (2006.01)
***B32B 27/30*** (2006.01) ***C07C 327/22*** (2006.01)
***C08F 220/20*** (2006.01) ***C08G 75/02*** (2016.01)
***G02B 1/04*** (2006.01)

(52) Cooperative Patent Classification (CPC):
**B32B 17/10; B32B 27/30; C07C 327/22; C08F 20/38; C08F 220/20; C08G 75/02; G02B 1/04**

(86) International application number:
**PCT/JP2023/005701**

(87) International publication number:
**WO 2023/188968 (05.10.2023 Gazette 2023/40)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.03.2022 JP 2022052197**

(71) Applicant: **Mitsui Chemicals, Inc.**
**Tokyo 104-0028 (JP)**

(72) Inventor: **TSUKADA Yuichi**
**Sodegaura-shi, Chiba 299-0265 (JP)**

(74) Representative: **Hoffmann Eitle**
**Patent- und Rechtsanwälte PartmbB**
**Arabellastraße 30**
**81925 München (DE)**

## (54) POLYFUNCTIONAL (METH)ACRYLATE THIOESTER COMPOSITION, CURABLE COMPOSITION, CURED PRODUCT, MOLDED BODY, OPTICAL MATERIAL, AND METHOD FOR PRODUCING POLYFUNCTIONAL (METH)ACRYLATE THIOESTER COMPOSITION

(57) Provided are [1] a polyfunctional (meth)acrylate thioester composition containing a polyfunctional (meth)acrylate thioester compound (A) represented by Formula (1), [2] a curable composition containing the polyfunctional (meth) acrylate thioester composition [1], [3] a cured product obtained by curing the curable composition [2], [4] a molded body including the cured product [3], and [5] an optical material including the molded body [4].

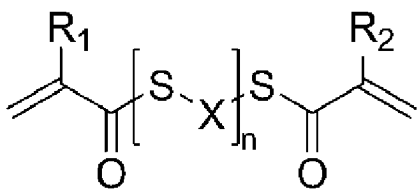

(1)

EP 4 501 978 A1

## Description

TECHNICAL FIELD

The present invention relates to a polyfunctional (meth)acrylate thioester composition, a curable composition, a cured product, a molded body, an optical material, and a method for producing a polyfunctional (meth)acrylate thioester composition.

BACKGROUND ART

In the related art, glass has been suitably used as an optical material because the glass has a diversity of refractive index and has a small fluctuation of refractive index due to temperature and humidity. However, in recent years, a resin cured product has been used in response to demands for weight reduction, cost reduction, and the like.

For example, a wafer-level lens has been used in a camera lens module mounted on a smartphone in order to reduce the size and the height. In the wafer-level lens, a resin cured product having a high refractive index and having excellent heat resistance is required. In addition, in a so-called hybrid type wafer-level lens in which a resin lens is formed on a glass substrate, in order to suppress peeling between the glass substrate and the resin lens due to residual stress, it is required that a curing shrinkage rate when a composition cures is low.

As a method for obtaining a resin cured product having a higher refractive index, a method of using a material having transparency, which is obtained by photo-curing a composition containing thio(meth)acrylate or the like, has been reported in Patent Document 1 and Patent Document 2.

RELATED DOCUMENT

PATENT DOCUMENT

[Patent Document 1] Pamphlet of International Publication No. WO1998/24761
[Patent Document 2] Japanese Unexamined Patent Publication No. H08-325337

SUMMARY OF THE INVENTION

TECHNICAL PROBLEM

However, the resin cured product obtained by the methods described in Patent Documents 1 and 2 has a high refractive index but has a large shrinkage rate in a case of being cured, and thus it has a problem in adhesiveness to glass.

The present invention has been made in view of the above circumstances, and an object of the present invention is to provide a polyfunctional (meth)acrylate thioester composition and a curable composition, with which a cured product with an improved performance balance of high refractive index, low curing shrinkage, and glass adhesiveness can be obtained; and a cured product, a molded body, and an optical material with an improved performance balance of high refractive index, low curing shrinkage, and glass adhesiveness.

SOLUTION TO PROBLEM

The present inventors have made intensive studies to solve the above-described problems. As a result, it has been found, by using a specific polyfunctional (meth)acrylate thioester compound, it is possible to improve the performance balance of high refractive index, low curing shrinkage, and glass adhesiveness of the cured product to be obtained, and thus the present invention has been completed.

According to the present invention, there are provided a polyfunctional (meth)acrylate thioester composition, a curable composition, a cured product, a molded body, an optical material, and a method for producing a polyfunctional (meth)acrylate thioester composition.

[1] A polyfunctional (meth)acrylate thioester composition containing:

a polyfunctional (meth)acrylate thioester compound (A) represented by Formula (1).

[Chem. 1]

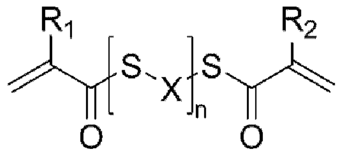

(1)

(in Formula (1), n represents an integer of 5 or more, X represents an alkylene group having 1 or more and 4 or less carbon atoms, in which any methylene group may be substituted with a carbonyl group, provided that any hydrogen atom in the alkylene group is substituted with a structure of Formula (2), and $R_1$ and $R_2$ each independently represent a hydrogen atom or a methyl group)

[Chem. 2]

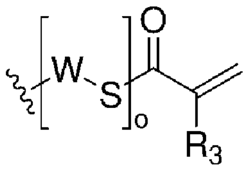

(2)

(in Formula (2), $R_3$ represents a hydrogen atom or a methyl group, a plurality of $R_3$'s may be the same or different from each other, W represents an alkylene group having 1 or more and 4 or less carbon atoms, and o represents an integer of 1 or more)

[2] The polyfunctional (meth)acrylate thioester composition according to [1],
in which a weight-average molecular weight of the polyfunctional (meth)acrylate thioester composition is 1,000 or more and 100,000 or less.
[3] The polyfunctional (meth)acrylate thioester composition according to [1] or [2],

in which the polyfunctional (meth)acrylate thioester compound (A) includes at least one selected from the group consisting of a compound represented by Formula (3) and a compound represented by Formula (4).

[Chem. 3]

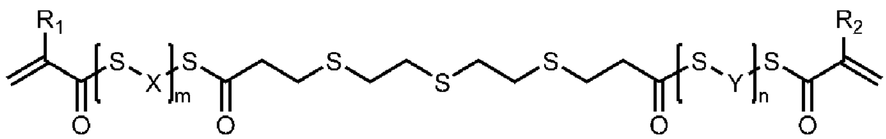

(3)

[Chem. 4]

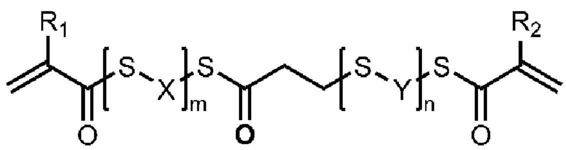

(4)

(in Formulae (3) and (4), m and n each independently represent an integer of 2 or more, X and Y each independently represent an alkylene group having 1 or more and 4 or less carbon atoms, in which any hydrogen atom may be substituted with the structure of Formula (2), a plurality of X's and a plurality of Y's may be each the same or different from each other, and $R_1$ and $R_2$ each independently represent a hydrogen atom or a methyl group)

[4] A curable composition containing:

the polyfunctional (meth)acrylate thioester composition according to any one of [1] to [3].
[5] The curable composition according to [4], further containing:

a compound (B) represented by Formula (5).

[Chem. 5]

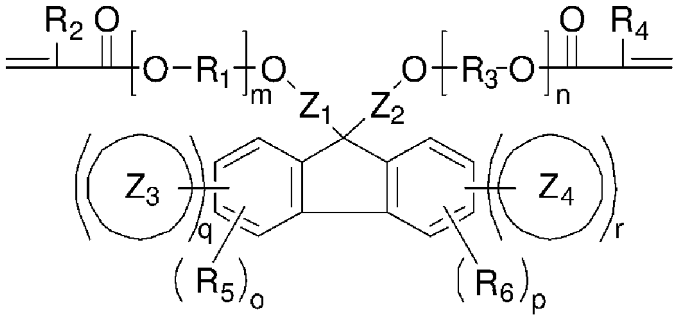

(5)

(in Formula (5), $Z_1$ and $Z_2$ each independently represent an aromatic carbon ring or an alkylene group, provided that any hydrogen atom may be substituted with an alkyl group or an alkoxy group, $Z_3$ and $Z_4$ each independently represent an aromatic carbon ring or an alkyl group, provided that any hydrogen atom may be substituted with an alkyl group or an alkoxy group, $R_1$ and $R_3$ each independently represent an alkylene group having 1 or more and 10 or less carbon atoms, $R_2$ and $R_4$ each independently represent a hydrogen atom or a methyl group, $R_5$ and $R_6$ each independently represent an alkyl group, m and n each independently represent an integer of 0 or more, and o, p, q, and r each represent an integer of 0 or more, satisfying relationships of $o + q \leq 4$ and $p + r \leq 4$)

[6] The curable composition according to [4] or [5],
in which a viscosity of the curable composition at 25°C, which is measured using an E-type viscometer under a condition of a rotation speed of 2.5 rpm, is 100 mPa·s or more and 10,000 mPa·s or less.
[7] The curable composition according to any one of [4] to [6], further containing:
a polymerization initiator.
[8] The curable composition according to any one of [4] to [7], further containing:
at least one selected from the group consisting of a silane coupling agent, an antioxidant, an ultraviolet absorber, and a light stabilizer.
[9] The curable composition according to any one of [4] to [8],
in which, in a case where a specific gravity of the curable composition, which is measured using a pycnometer in accordance with JIS Z 8804:2012, is denoted as $d^1$, and a specific gravity of a test piece having a thickness of 250 μm, which is formed of a cured product of the curable composition, is denoted as $d^2$, a curing shrinkage rate of the curable composition, which is represented by $(1 - d^1/d^2) \times 100$, is 9.0% or less.
[10] The curable composition according to any one of [4] to [9],
in which the curable composition is usable in an optical material.
[11] A cured product obtained by curing the curable composition according to any one of [4] to [10].
[12] The cured product according to [11],
in which, in a case where a test piece having a thickness of 250 μm is produced from the cured product, a light transmittance ($T_1$) of the test piece at a wavelength of 400 nm is 81% or more.
[13] The cured product according to [12],
in which, in a case where a light transmittance at a wavelength of 400 nm of the test piece after being heated in an oven set to 125°C in an air atmosphere for 168 hours is denoted as $T_2$, a rate of change in light transmittance after the heating, which is represented by $(T_1 - T_2)/T_1 \times 100$, is 7.0% or less.
[14] The cured product according to any one of [11] to [13],
in which, in a case where a test piece having a thickness of 250 μm is produced from the cured product, a refractive index (nD) of the test piece with respect to D-line (589.3 nm) is 1.600 or more.
[15] The cured product according to any one of [11] to [14],
in which, in a case where a test piece having a thickness of 250 μm is produced from the cured product, an Abbe number (νD) of the test piece, which is measured in accordance with ASTM D542, is 20 or more.
[16] A molded body including:
the cured product according to any one of [11] to [15].
[17] An optical material including:

the molded body according to [16].

[18] The optical material according to [17], further including:

a glass substrate,
in which the optical material is a laminate of the molded body and the glass substrate.

[19] The optical material according to [17] or [18],
in which the optical material is an optical lens.

[20] A method for producing the polyfunctional (meth)acrylate thioester composition according to any one of [1] to [3], the method including:

a step of converting, among total thiol groups in a polythiol compound, 0.5 equivalent or more and 0.9 equivalent or less of thiol groups into a functional group represented by the following structural formula and subjecting the obtained compound to a β-elimination reaction and an ene-thiol reaction to synthesize a polyfunctional (meth) acrylate thioester compound.

[Chem. 6]

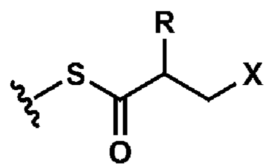

(in the structural formula, X represents a fluorine atom, a chlorine atom, a bromine atom, or an iodine atom, and R represents a hydrogen atom or a methyl group)

[21] A method for producing the polyfunctional (meth)acrylate thioester composition according to any one of [1] to [3], the method including:
a step of reacting a polythiol compound with a polythio(meth)acrylate compound by an ene-thiol reaction.

ADVANTAGEOUS EFFECTS OF INVENTION

According to the present invention, it is possible to provide a polyfunctional (meth)acrylate thioester composition and a curable composition, with which a cured product with an improved performance balance of high refractive index, low curing shrinkage, and glass adhesiveness can be obtained; and a cured product, a molded body, and an optical material with an improved performance balance of high refractive index, low curing shrinkage, and glass adhesiveness.

DESCRIPTION OF EMBODIMENTS

**Hereinafter,** the present invention will be described based on embodiments. In the present specification, "A to B" indicating a numerical range represents "A or more and B or less" unless otherwise specified. In addition, a notation of "(meth)acrylic" in the present specification indicates at least one selected from the group consisting of acrylic and methacrylic, and a similar notation such as "thio(meth)acrylate" and "(meth)acrylate" is also the same. In addition, "(meth) acryloyl group" in the present specification indicates at least one selected from the group consisting of an acryloyl group represented by $-C(=O)-CH=CH_2$ and a methacryloyl group represented by $-C(=O)-C(CH_3)=CH_2$.

[Polyfunctional (meth)acrylate thioester composition]

The polyfunctional (meth)acrylate thioester composition according to the present invention contains a polyfunctional (meth)acrylate thioester compound (A) represented by Formula (1) (hereinafter, also simply referred to as "compound (A)"). The polyfunctional (meth)acrylate thioester composition according to the present invention may contain only one kind of the polyfunctional (meth)acrylate thioester compound (A), or may contain two or more kinds of the polyfunctional (meth)acrylate thioester compounds (A). In a case where the polyfunctional (meth)acrylate thioester composition according to the present invention contains only one kind of the polyfunctional (meth)acrylate thioester compound (A), the polyfunctional (meth)acrylate thioester composition is also referred to as a polyfunctional (meth) acrylate thioester composition.

[Chem. 7]

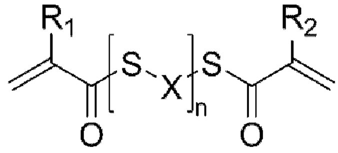

(1)

In Formula (1), n represents an integer of 5 or more, X represents an alkylene group having 1 or more and 4 or less carbon atoms, in which any methylene group may be substituted with a carbonyl group, provided that any hydrogen atom in the alkylene group is substituted with a structure of Formula (2), and $R_1$ and $R_2$ each independently represent a hydrogen atom or a methyl group.

[Chem. 8]

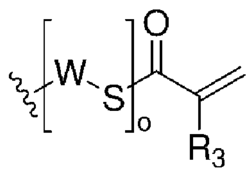

(2)

In Formula (2), $R_3$ represents a hydrogen atom or a methyl group, a plurality of $R_3$'s may be the same or different from each other, W represents an alkylene group having 1 or more and 4 or less carbon atoms, and o represents an integer of 1 or more.

In Formula (1), n represents an integer of 5 or more, preferably an integer of 6 or more and more preferably an integer of 7 or more, and preferably an integer of 20 or less, more preferably an integer of 15 or less, still more preferably an integer of 12 or less, and even more preferably an integer of 10 or less.

In Formula (1), X represents an alkylene group having 1 or more and 4 or less carbon atoms, more preferably an alkylene group having 2 or more and 4 or less carbon atoms, and still more preferably an alkylene group having 2 or 3 carbon atoms, in which any methylene group may be substituted with a carbonyl group, provided that any hydrogen atom in the alkylene group is substituted with the structure of Formula (2).

In Formula (1), the number of alkylene groups substituted with the structure of Formula (2) in X is preferably 1 or more and 4 or less, more preferably 1 or more and 3 or less, still more preferably 1 or 2, and even more preferably 2.

In Formula (1), the number of carbonyl groups in X is preferably 1 or more and 4 or less, more preferably 1 or more and 3 or less, and still more preferably 1 or 2.

In Formula (2), W represents an alkylene group having 1 or more and 4 or less carbon atoms, preferably an alkylene group having 1 or more and 3 or less carbon atoms, more preferably an alkylene group having 1 or 2 carbon atoms, and still more preferably an alkylene group having 1 carbon atom; and o represents an integer of 1 or more, preferably an integer of 1 or more and 4 or less, more preferably an integer of 1 or more and 3 or less, still more preferably 1 or 2, and even more preferably 1.

From the viewpoint of further improving the performance balance of high refractive index, low curing shrinkage, and glass adhesiveness of a cured product to be obtained, it is preferable that the compound (A) includes at least one selected from the group consisting of a compound represented by Formula (3) and a compound represented by Formula (4).

[Chem. 9]

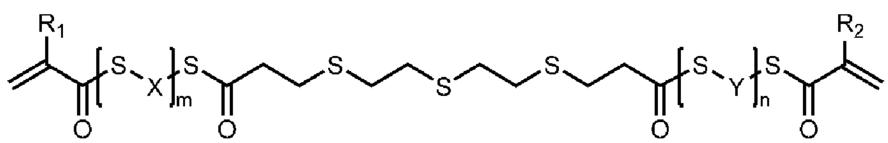

(3)

[Chem. 10]

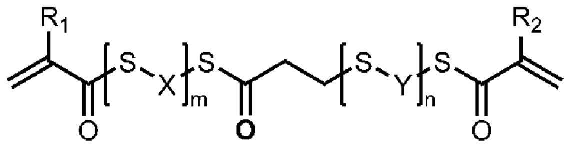

(4)

In Formulae (3) and (4), m and n each independently represent an integer of 2 or more, X and Y each independently represent an alkylene group having 1 or more and 4 or less carbon atoms, in which any hydrogen atom may be substituted with the structure of Formula (2), a plurality of X's and a plurality of Y's may be each the same or different from each other, and $R_1$ and $R_2$ each independently represent a hydrogen atom or a methyl group.

In Formulae (3) and (4), m and n each independently represent an integer of 2 or more, preferably an integer of 2 or more and 6 or less, more preferably an integer of 2 or more and 5 or less, still more preferably an integer of 2 or 3, and even more preferably 3.

In Formulae (3) and (4), X and Y each independently represent an alkylene group having 2 or more and 4 or less carbon atoms, in which any hydrogen atom may be substituted with the structure of Formula (2); preferably an alkylene group having 2 or 3 carbon atoms, in which any hydrogen atom may be substituted with the structure of Formula (2), and more preferably an alkylene group having 2 carbon atoms, in which any hydrogen atom may be substituted with the structure of Formula (2).

In Formulae (3) and (4), the number of alkylene groups substituted with the structure of Formula (2) in X and Y is preferably 1 or more and 4 or less, more preferably 1 or more and 3 or less, still more preferably 1 or 2, and even more preferably 2.

From the viewpoint of further improving the performance balance of high refractive index, low curing shrinkage, and glass adhesiveness of a cured product to be obtained, it is preferable that the compound (A) includes at least one selected from the group consisting of the following compounds.

[Chem. 11]

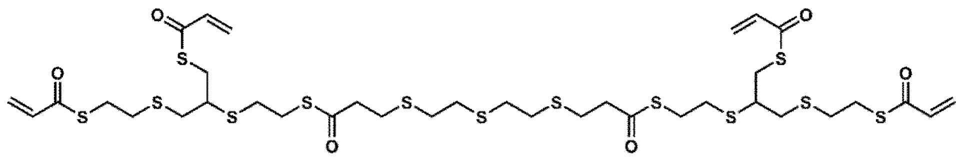

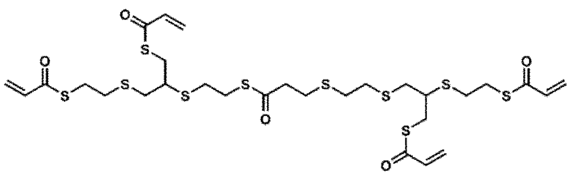

From the viewpoint of further improving the performance balance of high refractive index, low curing shrinkage, and glass adhesiveness of a cured product to be obtained, a weight-average molecular weight of the polyfunctional (meth) acrylate thioester composition according to the present invention is preferably 1,000 or more and more preferably 1,500 or more, and preferably 100,000 or less, more preferably 50,000 or less, still more preferably 20,000 or less, still more preferably 10,000 or less, and even more preferably 5,000 or less.

From the viewpoint of further improving the performance balance of high refractive index, low curing shrinkage, and glass adhesiveness of a cured product to be obtained, in a case where the total of the polyfunctional (meth)acrylate thioester composition is set to 100% by mass, a content of the compound (A) in the polyfunctional (meth)acrylate thioester composition according to the present invention is preferably 70% by mass or more, more preferably 80% by mass or more, still more preferably 90% by mass or more, and even still more preferably 95% by mass or more, and is preferably 100% by mass or less.

(Method for producing polyfunctional (meth)acrylate thioester composition)

The polyfunctional (meth)acrylate thioester composition according to the present invention can be produced by,

for example, a production method including a step of converting, among total thiol groups in a polythiol compound, 0.5 equivalent or more and 0.9 equivalent or less of thiol groups into a functional group represented by the following structural formula and subjecting the obtained compound to a β-elimination reaction and an ene-thiol reaction to synthesize a polyfunctional (meth)acrylate thioester compound.

More specifically, as in Synthesis Example 3 described later, the thiol group in the polythiol compound can be converted into the functional group represented by the following structural formula by reacting the polythiol compound with a halogenated alkylcarbonyl chloride compound such as 3-chloropropionyl chloride. The β-elimination reaction and the ene-thiol reaction can be carried out by known methods.

[Chem. 12]

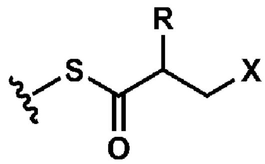

In the structural formula, X represents a fluorine atom, a chlorine atom, a bromine atom, or an iodine atom, preferably represents a chlorine atom, and R represents a hydrogen atom or a methyl group.

In addition, the polyfunctional (meth)acrylate thioester composition according to the present invention can also be produced, for example, by a production method including a step of reacting a polythiol compound with a polythio(meth)acrylate compound by an ene-thiol reaction.

More specifically, as in Synthesis Example 2 described later, the polyfunctional (meth)acrylate thioester composition can be obtained by reacting the polythio(meth)acrylate compound with the polythiol compound by an ene-thiol reaction. The ene-thiol reaction can be carried out by a known method.

[Curable composition]

The curable composition according to the present invention contains the above-described polyfunctional (meth)acrylate thioester composition according to the present invention.

With the curable composition according to the present invention, it is possible to obtain a cured product, a molded body, and an optical material in which a performance balance of high refractive index, low curing shrinkage, and glass adhesiveness is improved.

Hereinafter, each component constituting the curable composition according to the present invention will be described.

<Polyfunctional (meth)acrylate thioester composition>

The polyfunctional (meth)acrylate thioester composition is the polyfunctional (meth)acrylate thioester composition according to the present invention described above.

From the viewpoint of further improving the performance balance of high refractive index, low curing shrinkage, and glass adhesiveness of the cured product to be obtained, a content of the polyfunctional (meth)acrylate thioester composition in the curable composition according to the present invention is preferably 30 parts by mass or more, more preferably 40 parts by mass or more, still more preferably 50 parts by mass or more, even more preferably 70 parts by mass or more, even still more preferably 80 parts by mass or more, and further more preferably 90 parts by mass or more, and is preferably 100 parts by mass or less and more preferably 99 parts by mass or less, with respect to 100 parts by mass of the curable composition according to the present invention.

<Compound (B)>

From the viewpoint of further reducing Abbe number and viewpoint of further improving transparency, heat resistance, and stability after heating, the curable composition according to the present invention can further contain a compound (B) represented by Formula (5) (hereinafter, also simply referred to as "compound (B)").

[Chem. 13]

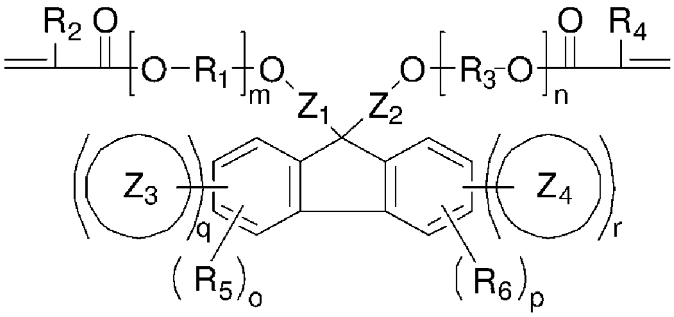

(5)

In Formula (5), $Z_1$ and $Z_2$ each independently represent an aromatic carbon ring or an alkylene group, provided that any hydrogen atom may be substituted with an alkyl group or an alkoxy group, $Z_3$ and $Z_4$ each independently represent an aromatic carbon ring or an alkyl group, provided that any hydrogen atom may be substituted with an alkyl group or an alkoxy group, $R_1$ and $R_3$ each independently represent an alkylene group having 1 or more and 10 or less carbon atoms, $R_2$ and $R_4$ each independently represent a hydrogen atom or a methyl group, $R_5$ and $R_6$ each independently represent an alkyl group, m and n each independently represent an integer of 0 or more, and o, p, q, and r each represent an integer of 0 or more, satisfying relationships of $o + q \leq 4$ and $p + r \leq 4$.

In Formula (5), $Z_1$ and $Z_2$ each independently represent an aromatic carbon ring or an alkylene group, provided that any hydrogen atom may be substituted with an alkyl group or an alkoxy group. $Z_1$ and $Z_2$ each independently represent preferably a benzene ring, a naphthalene ring, or an alkylene group having 1 or more and 4 or less carbon atoms, and more preferably a benzene ring or an alkylene group having 1 or more and 3 or less carbon atoms.

In Formula (5), $Z_3$ and $Z_4$ each independently represent an aromatic carbon ring or an alkyl group, provided that any hydrogen atom may be substituted with an alkyl group or an alkoxy group. $Z_3$ and $Z_4$ each independently represent preferably an aromatic carbon ring, more preferably a benzene ring or a naphthalene ring, and still more preferably a naphthalene ring.

In Formula (5), $R_1$ and $R_3$ each independently represent an alkylene group having 1 or more and 10 or less carbon atoms. $R_1$ and $R_3$ each independently represent preferably an alkylene group having 1 or more and 4 or less carbon atoms, more preferably an alkylene group having 1 or more and 3 or less carbon atoms, still more preferably an alkylene group having 2 or 3 carbon atoms, and even more preferably an alkylene group having 2 carbon atoms.

In Formula (5), $R_2$ and $R_4$ each independently represent a hydrogen atom or a methyl group, $R_2$ and $R_4$ each independently represent preferably a hydrogen atom.

In Formula (5), $R_5$ and $R_6$ each independently represent an alkyl group. $R_s$ and $R_6$ each independently represent preferably an alkyl group having 1 or more and 4 or less carbon atoms, more preferably represent an alkyl group having 1 or 2 carbon atoms, and still more preferably represent an alkyl group having 1 carbon atom.

In Formula (5), m and n each independently represent an integer of 0 or more. m and n each independently represent preferably an integer of 0 or more and 4 or less, more preferably an integer of 0 or more and 2 or less, still more preferably 0 or 1, and even more preferably 1.

In Formula (5), o, p, q, and r each represent an integer of 0 or more, satisfying relationships of $o + q \leq 4$ and $p + r \leq 4$. q and r each independently represent preferably an integer of 0 or more and 2 or less, more preferably 0 or 1, and still more preferably 0. o and p each independently represent preferably an integer of 0 or more and 2 or less, more preferably 0 or 1, and still more preferably 0.

From the viewpoint of further improving the performance balance of high refractive index, low curing shrinkage, and glass adhesiveness of the cured product to be obtained, the compound (B) is preferably the compound represented by Formula (5), in which $Z_1$ and $Z_2$ represent a benzene ring, a naphthalene ring, or an alkylene group having 1 or more and 4 or less carbon atoms, $Z_3$ and $Z_4$ represent a benzene ring or a naphthalene ring, $R_1$ and $R_3$ are an alkylene group having 1 or more and 4 or less carbon atoms, $R_2$ and $R_4$ represent a hydrogen atom or a methyl group, m and n represent an integer of 0 or more and 4 or less, q and r represent 0 or 1, and o and p represent 0; and more preferably the compound represented by Formula (5), in which $Z_1$ and $Z_2$ represent a benzene ring or an alkylene group having 1 or more and 3 or less carbon atoms, $Z_3$ and $Z_4$ represent a naphthalene ring, $R_1$ and $R_3$ represent an alkylene group having 1 or more and 3 or less carbon atoms, $R_2$ and $R_4$ represent a hydrogen atom or a methyl group, m and n represent 0 or 1, q and r represent 0 or 1, and o and p represent 0.

From the viewpoint of further improving the performance balance of high refractive index, low curing shrinkage, and glass adhesiveness of the cured product to be obtained, the compound (B) is preferably at least one selected from the group consisting of 9,9-bis(4-((meth)acryloyloxyphenyl)fluorene, 9,9-bis(4-(2-(meth)acryloyloxyethoxy)phenyl)fluorene, 9,9-bis(4-(3-(meth)acryloyloxypropoxy)phenyl)fluorene, 9,9-bis(4-(2-(meth)acryloyloxypropoxy)phenyl)fluorene, 9,9-bis(4-(meth)acryloyloxy-3-methylphenyl)fluorene, 9,9-bis[4-(2-(meth)acryloyloxyethoxy)-3-methylphenyl]fluorene, 9,9-bis[4-(3-(meth)acryloyloxypropoxy)-3-methylphenyl]fluorene, 9,9-bis[4-(2-(meth)acryloyloxypropoxy)-3-methylphenyl]fluorene, 9,9-bis(4-(meth)acryloyloxy-3-ethylphenyl)fluorene, 9,9-bis[4-(2-(meth)acryloyloxyethoxy)-3-ethylphenyl] fluorene, 9,9-bis[4-(3-(meth)acryloyloxypropoxy)-3-ethylphenyl]fluorene, 9,9-bis[4-(2-(meth)acryloyloxypropoxy)-3-ethylphenyl]fluorene, 9,9-bis(2-(meth)acryloyloxyethyl)-1,8-diphenylfluorene, 9,9-bis(3-(meth)acryloyloxypropyl)-1,8-diphenylfluorene, 9,9-bis(2-(meth)acryloyloxypropyl)-1,8-diphenylfluorene, 9,9-bis(2-(meth)acryloyloxyethyl)-2,7-diphenylfluorene, 9,9-bis(3-(meth)acryloyloxypropyl)-2,7-diphenylfluorene, 9,9-bis(2-(meth)acryloyloxypropyl)-2,7-diphenylfluorene, 9,9-bis(2-(meth)acryloyloxyethyl)-3,6-diphenylfluorene, 9,9-bis(3-(meth)acryloyloxypropyl)-3,6-diphenylfluorene, 9,9-bis(2-(meth)acryloyloxypropyl)-3,6-diphenylfluorene, 9,9-bis(2-(meth)acryloyloxyethyl)-4,5-diphenylfluorene, 9,9-bis(3-(meth)acryloyloxypropyl)-4,5-diphenylfluorene, 9,9-bis(2-(meth)acryloyloxypropyl)-4,5-diphenylfluorene, 9,9-bis(2-(meth)acryloyloxyethyl)-1,8-bis(naphth-1-yl)fluorene, 9,9-bis(3-(meth)acryloyloxypropyl)-1,8-bis(naphth-1-yl) fluorene, 9,9-bis(2-(meth)acryloyloxypropyl)-1,8-bis(naphth-1-yl)fluorene, 9,9-bis(2-(meth)acryloyloxyethyl)-2,7-bis(naphth-1-yl)fluorene, 9,9-bis(3-(meth)acryloyloxypropyl)-2,7-bis(naphth-1-yl)fluorene, 9,9-bis(2-(meth)acryloyloxypropyl)-2,7-bis(naphth-1-yl)fluorene, 9,9-bis(2-(meth)acryloyloxyethyl)-3,6-bis(naphth-1-yl)fluorene, 9,9-bis(3-(meth) acryloyloxypropyl)-3,6-bis(naphth-1-yl)fluorene, 9,9-bis(2-(meth)acryloyloxypropyl)-3,6-bis(naphth-1-yl)fluorene, 9,9-bis(2-(meth)acryloyloxyethyl)-4,5-bis(naphth-1-yl)fluorene, 9,9-bis(3-(meth)acryloyloxypropyl)-4,5-bis(naphth-1-yl) fluorene, 9,9-bis(2-(meth)acryloyloxypropyl)-4,5-bis(naphth-1-yl)fluorene, 9,9-bis(2-(meth)acryloyloxyethyl)-1,8-bis(naphth-2-yl)fluorene, 9,9-bis(3-(meth)acryloyloxypropyl)-1,8-bis(naphth-2-yl)fluorene, 9,9-bis(2-(meth)acryloyloxypropyl)-1,8-bis(naphth-2-yl)fluorene, 9,9-bis(2-(meth)acryloyloxyethyl)-2,7-bis(naphth-2-yl)fluorene, 9,9-bis(3-(meth) acryloyloxypropyl)-2,7-bis(naphth-2-yl)fluorene, 9,9-bis(2-(meth)acryloyloxypropyl)-2,7-bis(naphth-2-yl)fluorene, 9,9-bis(2-(meth)acryloyloxyethyl)-3,6-bis(naphth-2-yl)fluorene, 9,9-bis(3-(meth)acryloyloxypropyl)-3,6-bis(naphth-2-yl) fluorene, 9,9-bis(2-(meth)acryloyloxypropyl)-3,6-bis(naphth-2-yl)fluorene, 9,9-bis(2-(meth)acryloyloxyethyl)-4,5-bis(naphth-2-yl)fluorene, 9,9-bis(3-(meth)acryloyloxypropyl)-4,5-bis(naphth-2-yl)fluorene, and 9,9-bis(2-(meth)acryloyloxypropyl)-4,5-bis(naphth-2-yl)fluorene; more preferably at least one selected from the group consisting of 9,9-bis(4-(meth)acryloyloxyphenyl)fluorene, 9,9-bis(4-(2-(meth)acryloyloxyethoxy)phenyl)fluorene, 9,9-bis(4-(3-(meth)acryloyloxypropoxy)phenyl)fluorene, 9,9-bis(4-(2-(meth)acryloyloxypropoxy)phenyl)fluorene, 9,9-bis(2-(meth)acryloyloxyethyl)-2,7-diphenylfluorene, 9,9-bis(3-(meth)acryloyloxypropyl)-2,7-diphenylfluorene, 9,9-bis(2-(meth)acryloyloxypropyl)-2,7-diphenylfluorene, 9,9-bis(2-(meth)acryloyloxyethyl)-3,6-diphenylfluorene, 9,9-bis(3-(meth)acryloyloxypropyl)-3,6-diphenylfluorene, 9,9-bis(2-(meth)acryloyloxypropyl)-3,6-diphenylfluorene, 9,9-bis(2-(meth)acryloyloxyethyl)-2,7-bis(naphth-1-yl)fluorene, 9,9-bis(3-(meth)acryloyloxypropyl)-2,7-bis(naphth-1-yl)fluorene, 9,9-bis(2-(meth) acryloyloxypropyl)-2,7-bis(naphth-1-yl)fluorene, 9,9-bis(2-(meth)acryloyloxyethyl)-3,6-bis(naphth-1-yl)fluorene, 9,9-bis(3-(meth)acryloyloxypropyl)-3,6-bis(naphth-1-yl)fluorene, 9,9-bis(2-(meth)acryloyloxypropyl)-3,6-bis(naphth-1-yl) fluorene, 9,9-bis(2-(meth)acryloyloxyethyl)-2,7-bis(naphth-2-yl)fluorene, 9,9-bis(3-(meth)acryloyloxypropyl)-2,7-bis(naphth-2-yl)fluorene, 9,9-bis(2-(meth)acryloyloxypropyl)-2,7-bis(naphth-2-yl)fluorene, 9,9-bis(2-(meth)acryloyloxyethyl)-3,6-bis(naphth-2-yl)fluorene, 9,9-bis(3-(meth)acryloyloxypropyl)-3,6-bis(naphth-2-yl)fluorene, and 9,9-bis(2-(meth)acryloyloxypropyl)-3,6-bis(naphth-2-yl)fluorene; and still more preferably at least one selected from the group consisting of 9,9-bis(4-(2-(meth)acryloyloxyethoxy)phenyl)fluorene, 9,9-bis(4-(3-(meth)acryloyloxypropoxy)phenyl)fluorene, 9,9-bis(4-(2-(meth)acryloyloxypropoxy)phenyl)fluorene, 9,9-bis(2-(meth)acryloyloxyethyl)-2,7-bis(naphth-1-yl)fluorene, 9,9-bis(3-(meth)acryloyloxypropyl)-2,7-bis(naphth-1-yl)fluorene, 9,9-bis(2-(meth)acryloyloxypropyl)-2,7-bis(naphth-1-yl)fluorene, 9,9-bis(2-(meth)acryloyloxyethyl)-3,6-bis(naphth-1-yl)fluorene, 9,9-bis(3-(meth) acryloyloxypropyl)-3,6-bis(naphth-1-yl)fluorene, 9,9-bis(2-(meth)acryloyloxypropyl)-3,6-bis(naphth-1-yl)fluorene, 9,9-bis(2-(meth)acryloyloxyethyl)-2,7-bis(naphth-2-yl)fluorene, 9,9-bis(3-(meth)acryloyloxypropyl)-2,7-bis(naphth-2-yl) fluorene, 9,9-bis(2-(meth)acryloyloxypropyl)-2,7-bis(naphth-2-yl)fluorene, 9,9-bis(2-(meth)acryloyloxyethyl)-3,6-bis(naphth-2-yl)fluorene, 9,9-bis(3-(meth)acryloyloxypropyl)-3,6-bis(naphth-2-yl)fluorene, and 9,9-bis(2-(meth)acryloyloxypropyl)-3,6-bis(naphth-2-yl)fluorene.

From the viewpoint of further improving the performance balance of high refractive index, low Abbe number, low curing shrinkage, and glass adhesiveness of the cured product to be obtained, and viewpoint of further improving the transparency, the heat resistance, and the stability after heating, a content of the compound (B) in the curable composition according to the present invention is preferably 1 part by mass or more, more preferably 5 parts by mass or more, still more preferably 10 parts by mass or more, even more preferably 15 parts by mass or more, and even still more preferably 20 parts by mass or more, and is preferably 70 parts by mass or less, more preferably 60 parts by mass or less, and still more preferably 50 parts by mass or less, with respect to 100 parts by mass of the curable composition according to the present invention.

(Method of synthesizing compound (B))

Examples of a commercially available product of the compound (B) include a bifunctional acrylate "A-BPEF" manufactured by Shin-Nakamura Chemical Co., Ltd., a bifunctional acrylate "OGSOL EA-0200" and "OGSOL EA-0300" manufactured by Osaka Gas Chemicals Co., Ltd.

In addition, the compound (B) can be synthesized by subjecting a commercially available fluorene derivative diol

to (meth)acryloylation with (meth)acrylic acid anhydride, (meth)acryloyl chloride, or the like.

Examples of the commercially available fluorene derivative diol include bisphenoxyethanol fluorene (BPEF) or bisphenol fluorene (BPF) manufactured by Osaka Gas Chemicals Co., Ltd.

In addition, 9,9-bis(2-((meth)acryloyloxyethyl)-2,7-bis(naphth-2-yl)fluorene (DNEOA) can be synthesized by the following synthesis scheme.

[Chem. 14]

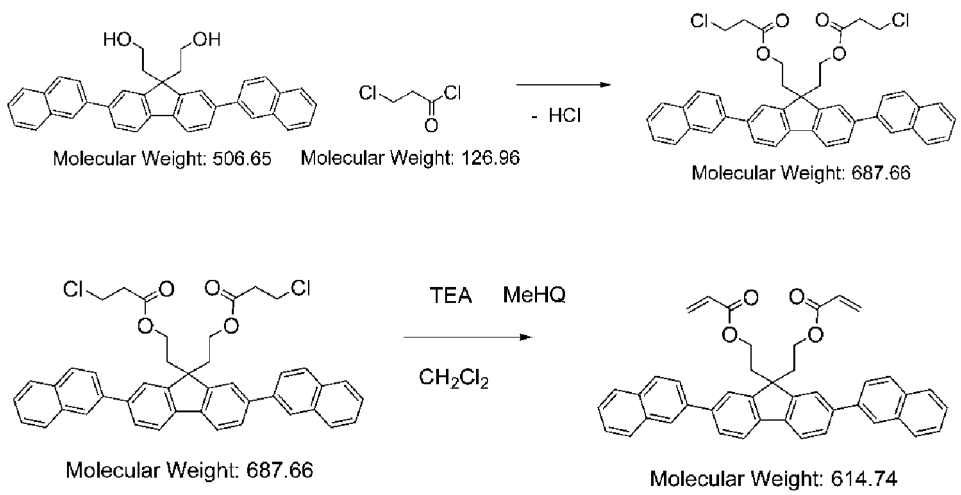


From the viewpoint of further improving the performance balance of high refractive index, low curing shrinkage, and glass adhesiveness of the cured product to be obtained, a viscosity of the curable composition according to the present invention at 25°C, which is measured using an E-type viscometer under a condition of a rotation speed of 2.5 rpm, is preferably 100 mPa·s or more, more preferably 500 mPa·s or more, still more preferably 1,000 mPa·s or more, and still more preferably 1,500 mPa·s or more, and is preferably 10,000 mPa·s or less, more preferably 8,000 mPa·s or less, still more preferably 6,500 mPa·s or less, still more preferably 5,000 mPa·s or less, and even more preferably 3,500 mPa·s or less.

<Other components>

The curable composition according to the present invention may contain other components in addition to the polyfunctional (meth)acrylate thioester composition and the compound (B). Examples of the other components include a polymerization initiator, an ultraviolet absorber, a resin modifier, an internal mold release agent, a silane coupling agent, an antioxidant, a light stabilizer, a processing stabilizer, a bluing agent, a polymerized metal deactivator, a flame retardant, a lubricant, an antistatic agent, a heat-ray shielding agent, a fluorescent dye (including a fluorescent brightener), a pigment, a light scattering agent, a reinforcing filler, a surfactant, an antibacterial agent, a plasticizer, a compatibilizer, and other resins or elastomers. Among these, from the viewpoint of further improving curing properties, the curable composition according to the present invention preferably contains a polymerization initiator. In addition, the curable composition according to the present invention preferably further contains at least one selected from the group consisting of a silane coupling agent, an antioxidant, an ultraviolet absorber, and a light stabilizer.

From the viewpoint of further improving the performance balance of high refractive index, low curing shrinkage, and glass adhesiveness of the cured product to be obtained, the curable composition according to the present invention preferably contains a polymerization initiator. As the polymerization initiator, for example, a thermal radical polymerization initiator, a photoradical polymerization initiator, or a combination thereof can be used.

Examples of the thermal radical polymerization initiator include dialkyl peroxides such as dicumyl peroxide, t-butyl cumyl peroxide, 2,5-bis(t-butylperoxy)2,5-dimethylhexane, 2,5-bis(t-butylperoxy)2,5-dimethylhexane-3, di-t-butyl peroxide, isopropylcumyl-t-butyl peroxide, and bis(α-t-butylperoxyisopropyl) benzene; peroxyketals such as n-butyl-4,4-bis(t-butylperoxy)valerate, 1,1-bis(t-butylperoxy)cyclohexane, 1,1-bis(t-butylperoxy)3,3,5-trimethylcyclohexane, 1,1-bis(t-butylperoxy)cyclododecane, ethyl-3,3-bis(t-butylperoxy)butyrate and 3,3,6,6,9,9-hexamethyl-1,2,4,5-tetraoxacyclononane; peroxyesters such as bis(t-butylperoxy)isophthalate, t-butylperoxybenzoate, and t-butylperoxyacetate; hydroperoxides such as t-butyl hydroperoxide, t-hexyl hydroperoxide, cumene hydroperoxide, 1,1,3,3-tetramethylbutyl hydroperoxide, diisopropylbenzene hydroperoxide, and p-mentane hydroperoxide; dibenzyl compounds such as 2,3-dimethyl-2,3-diphenylbutane; and 3,3,5,7,7-pentamethyl-1,2,4-trioxepane.

Examples of the photoradical polymerization initiator include benzoin alkyl ether, benzyl dimethyl ketal, 1-hydroxycyclohexyl phenyl ketone, 2-hydroxy-2-methyl-1-phenylpropan-1-one, benzophenone, methyl benzoyl formate, isopropyl thioxantone, and a mixture of two or more thereof. In addition, it is also possible to use a sensitizer together with these photoradical polymerization initiators. Examples of the sensitizer include carbonyl compounds such as anthraquinone, 1,2-naphthoquinone, 1,4-naphthoquinone, benzanthrone, p,p'-tetramethylbenzophenone, and chloranil; nitro compounds such as nitrobenzene, p-dinitrobenzene, and 2-nitrofluorene; aromatic hydrocarbons such as anthracene and chrysene; sulfur compounds such as diphenyldisulfide; and nitrogen compounds such as nitroaniline, 2-chloro-4-nitroaniline, 5-nitro-2-aminotoluene, and tetracyanoethylene.

From the viewpoint of further improving the performance balance of high refractive index, low curing shrinkage, and glass adhesiveness of the cured product to be obtained, in a case where the total amount of the polyfunctional (meth) acrylate thioester composition and the compound (B) in the curable composition according to the present invention is set to 100 parts by mass, a content of the polymerization initiator in the curable composition according to the present invention is preferably 0.1 parts by mass or more, more preferably 0.5 parts by mass or more, still more preferably 1.0 part by mass or more, even more preferably 1.5 parts by mass or more, even still more preferably 2.0 parts by mass or more, and further more preferably 2.5 parts by mass or more, and is preferably 10 parts by mass or less, more preferably 8.0 parts by mass or less, still more preferably 5.0 parts by mass or less, and even more preferably 4.0 parts by mass or less.

From the viewpoint of further improving the performance balance of high refractive index, low curing shrinkage, and glass adhesiveness of the cured product to be obtained, the curable composition according to the present invention preferably contains a silane coupling agent. Examples of the silane coupling agent include vinyltrimethoxysilane, vinyltriethoxysilane, 2-(3,4-epoxycyclohexyl)ethyltrimethoxysilane, 3-glycidoxypropylmethyldimethoxysilane, 3-glycidoxypropyltrimethoxysilane, 3-glycidoxypropylmethyldiethoxysilane, 3-glycidoxypropyltriethoxysilane, p-styryltrimethoxysilane, 3-methacryloxypropylmethyldimethoxysilane, 3-methacryloxypropyltrimethoxysilane, 3-methacryloxypropyltriethoxysilane, 3-acryloxypropyltrimethoxysilane, N-2-(aminoethyl)-3-aminopropylmethyldimethoxysilane, N-2-(aminoethyl)-3-aminopropyltrimethoxysilane, 3-aminopropyltrimethoxysilane, 3-aminopropyltriethoxysilane, 3-triethoxysilyl-N-(1,3-dimethylbutylidene)propylamine, N-phenyl-3-aminopropyltrimethoxysilane, N-(vinylbenzyl)-2-aminoethyl-3-aminopropyltrimethoxysilane hydrochloride, tris-(trimethoxysilylpropyl)isocyanurate, 3-ureidopropyltrialkoxysilane, 3-mercaptopropylmethyldimethoxysilane, 3-mercaptopropyltrimethoxysilane, 3-isocyanatopropyltriethoxysilane, and 3-trimethoxysilylpropionic acid anhydride. These silane coupling agents are easily available from the market because they are sold by, for example, Shin-Etsu Chemical Co., Ltd., or the like.

From the viewpoint of further improving the performance balance of high refractive index, low curing shrinkage, and glass adhesiveness of the cured product to be obtained, in a case where the total amount of the polyfunctional (meth) acrylate thioester composition and the compound (B) in the curable composition according to the present invention is set to 100 parts by mass, a content of the silane coupling agent in the curable composition according to the present invention is preferably 0.001 parts by mass or more, more preferably 0.01 parts by mass or more, still more preferably 0.1 part by mass or more, and even more preferably 0.5 parts by mass or more, and is preferably 10 parts by mass or less, more preferably 5.0 parts by mass or less, still more preferably 3.0 parts by mass or less, and even more preferably 1.5 parts by mass or less.

From the viewpoint of further improving ultraviolet resistance, the curable composition according to the present invention preferably contains an ultraviolet absorber. Examples of the ultraviolet absorber include a benzophenone-based ultraviolet absorber, a benzotriazole-based ultraviolet absorber, a triazine-based ultraviolet absorber, and a benzoxazine-based ultraviolet absorber. Examples of the benzophenone-based ultraviolet absorber include 4-methoxy-2-hydroxybenzophenone (molecular weight: 228), 4-methoxy-2-hydroxybenzophenone-5-sulfonic acid (molecular weight: 308), 2,4-dihydroxybenzophenone (molecular weight: 214), 4,4'-dimethoxy-2,2'-dihydroxybenzophenone (molecular weight: 274), 4,4'-dimethoxy-2,2'-dihydroxy-5,5'-disulfonic acid benzophenone disodium salt (molecular weight: 478), 2,2'-4,4'-tetrahydroxybenzophenone (molecular weight: 246), sodium hydroxymethoxybenzophenone sulfonate (molecular weight: 376), octabenzone (molecular weight: 326), 2-hydroxy-4-m-octyloxy-benzophenone (molecular weight: 345), 2-hydroxy-4-n-octyloxybenzophenone (molecular weight: 326), and bis(5-benzoyl-4-hydroxy-2-methoxyphenyl) methane (molecular weight: 468).

In addition, examples of the benzotriazole-based ultraviolet absorber include 2-(2H-benzotriazole-2-yl)-p-cresol (molecular weight: 225), 2-(2H-benzotriazole-2-yl)-4-6-bis(1-methyl-1-phenylethyl)phenol (molecular weight: 448), 2-[5-chloro(2H)-benzotriazole-2-yl]-4-methyl-6-(tert-butyl)phenol (molecular weight: 316), 2,4-di-tert-butyl-6-(5-chloro-2H-1,2,3-benzotriazole-2-yl)phenol (molecular weight: 358), 2-(2H-benzotriazole-2-yl)-4,6-tert-pentylphenol (molecular weight: 352), 2-(2H-benzotriazole-2-yl)-4-(1,1,3,3-tetramethylbutyl)phenol (molecular weight: 323), 2,2'-methylenebis [6-(2H-benzotriazole-2-yl)-4-(1,1,3,3-tetramethylbutyl)phenol] (molecular weight: 659), 2-(2'-hydroxy-3'-tert-butyl-5'-methylphenyl)-5-chlorobenzotriazole, 2-(2'-hydroxy-3',5-di-tert-butyl-phenyl)-5-chlorobenzotriazole (molecular weight: 357), 2-(2'-hydroxy-5'-methylphenyl)benzotriazole (molecular weight: 225), and 2-(2-hydroxy-5-octylphenyl)-benzotriazole (molecular weight: 323).

Furthermore, examples of the triazine-based ultraviolet absorber include 2-(4,6-diphenyl-1,3,5-triazin-2-yl)-5-[(hexyl)oxy]-phenol (molecular weight: 426), 2-[4,6-bis(2,4-dimethylphenyl)-1,3,5-triazin-2-yl]-5-(octyloxy)phenol (molecular weight: 509), 2,4,6-tris(2-hydroxy-4-hexyloxy-3-methylphenyl)-1,3,5-triazine (molecular weight: 700), 2-(4,6-diphenyl-1,3,5-triazin-2-yl)-5-[2-(2-ethylhexanoyloxy)ethoxy]phenol (molecular weight: 512), and 1,6-hexanediamine, N,N'-bis(1,2,2,6,6-pentamethyl-4-piperidyl), polymersmorpholine-2,4,6-trichloro-1,3,5-triazine.

In addition, examples of the benzoxazine-based ultraviolet absorber include 2,2'-(1,4-phenylene)bis(4H-3,1-benzoxazine-4-one) (molecular weight: 368). In addition, examples thereof include tetraethyl-2,2-(1,4-phenylene-dimethylene-bismalonate (molecular weight: 418) having a malonate ester structure and 2-ethyl-2'-ethoxy-oxamide (molecular weight: 312) having a succinimide structure. Two or more of the above-described components can be used in combination.

From the viewpoint of further improving the performance balance of high refractive index, low curing shrinkage, glass adhesiveness, and ultraviolet resistance of the cured product to be obtained, in a case where the total amount of the polyfunctional (meth)acrylate thioester composition and the compound (B) in the curable composition according to the present invention is set to 100 parts by mass, a content of the ultraviolet absorber in the curable composition according to the present invention is preferably 0.001 parts by mass or more, more preferably 0.005 parts by mass or more, and still more preferably 0.01 part by mass or more, and is preferably 1.0 part by mass or less, more preferably 0.5 parts by mass or less, still more preferably 0.2 parts by mass or less, and even more preferably 0.1 parts by mass or less.

From the viewpoint of further improving oxidation resistance, the curable composition according to the embodiment of the present invention preferably contains an antioxidant. Examples of the antioxidant include triethylene glycol-bis[3-(3-tert-butyl-5-methyl-4-hydroxyphenyl)propionate], 1,6-hexanediol-bis[3-(3,5-di-tert-butyl-4-hydroxyphenyl)propionate], pentaerythritol-tetrakis[3-(3,5-di-tert-butyl-4-hydroxyphenyl)propionate], octadecyl-3-(3,5-di-tert-butyl-4-hydroxyphenyl)propionate, 1,3,5-trimethyl-2,4,6-tris(3,5-di-tert-butyl-4-hydroxybenzyl)benzene, N,N-hexamethylenebis(3,5-di-tert-butyl-4-hydroxy-hydrocinnamide), 3,5-di-tert-butyl-4-hydroxy-benzylphosphonate-diethyl ester, tris(3,5-di-tert-butyl-4-hydroxybenzyl)isocyanurate, and 3,9-bis{1,1-dimethyl-2-[β-(3-tert-butyl-4-hydroxy-5-methylphenyl)propionyloxy]ethyl}-2,4,8,10-tetraoxaspiro(5,5)undecane.

From the viewpoint of further improving the performance balance of high refractive index, low curing shrinkage, glass adhesiveness, and oxidation resistance of the cured product to be obtained, in a case where the total amount of the polyfunctional (meth)acrylate thioester composition and the compound (B) in the curable composition according to the present invention is set to 100 parts by mass, a content of the antioxidant in the curable composition according to the present invention is preferably 0.01 parts by mass or more, more preferably 0.05 parts by mass or more, and still more preferably 0.1 part by mass or more, and is preferably 5.0 parts by mass or less, more preferably 2.0 parts by mass or less, and still more preferably 1.0 part by mass or less.

From the viewpoint of further improving weather resistance, the curable composition according to the present invention preferably contains a light stabilizer, and more preferably contains a hindered amine-based light stabilizer. Examples of the hindered amine-based light stabilizer include (1,2,2,6,6-pentamethyl-piperidin-4-yl) methacrylic acid, decanedioic acid bis(2,2,6,6-tetramethyl-1(octyloxy)-4-piperidinyl) ester, 1,1-dimethylethyl hydroperoxide, a reaction product of 70% by weight of octane and 30% by weight of polypropylene, a mixture of bis(1,2,2,6,6-pentamethyl-4-piperidyl) [[3,5-bis(1,1-dimethylethyl)-4-hydroxyphenyl]methyl]butyl malonate, bis(1,2,2,6,6-pentamethyl-4-piperidyl)sebacate, and methyl-1,2,2,6,6-pentamethyl-4-piperidyl sebacate, a mixture of bis(2,2,6,6-tetramethyl-4-piperidyl)sebacate, tetrakis(2,2,6,6-tetramethyl-4-piperidyl)-1,2,3,4-butanetetracarboxylate, tetrakis(1,2,2,6,6-pentamethyl-4-piperidyl)-1,2,3,4-butanetetracarboxylate, 2,2,6,6-tetramethyl-4-piperidyl-1,2,3,4-butanetetracarboxylate, and tridecyl-1,2,3,4-butanetetracarboxylate; and a mixture of 1,2,2,6,6-pentamethyl-4-piperidyl-1,2,3,4-butanetetracarboxylate and tridecyl-1,2,3,4-butanetetracarboxylate.

From the viewpoint of further improving the performance balance of high refractive index, low curing shrinkage, glass adhesiveness, and weather resistance of the cured product to be obtained, in a case where the total amount of the polyfunctional (meth)acrylate thioester composition and the compound (B) in the curable composition according to the present invention is set to 100 parts by mass, a content of the hindered amine-based light stabilizer in the curable composition according to the present invention is preferably 0.01 parts by mass or more, more preferably 0.03 parts by mass or more, and still more preferably 0.05 parts by mass or more, and is preferably 5.0 parts by mass or less, more preferably 1.0 part by mass or less, still more preferably 0.5 parts by mass or less, and even more preferably 0.2 parts by mass or less.

In the curable composition according to the present invention, in a case where a specific gravity of the curable composition, which is measured using a pycnometer in accordance with JIS Z 8804:2012, is denoted as $d^1$, and a specific gravity of a test piece having a thickness of 250 μm, which is formed of a cured product of the curable composition according to the present invention, is denoted as $d^2$, a curing shrinkage rate of the curable composition, which is represented by $(1 - d^1/d^2) \times 100$, is preferably 9.0% or less, more preferably 8.0% or less, still more preferably 7.0% or less, and even more preferably 6.5% or less. The lower limit value of the curing shrinkage rate is not particularly limited, but is, for example, 0.1% or more, and may be 1.0% or more, 3.0% or more, 4.0% or more, or 5.0% or more.

In the present embodiment, the test piece having a thickness of 250 μm, which is formed of the cured product of

the curable composition according to the present invention, can be obtained, for example, by irradiating a curable film formed of the curable composition applied onto a glass substrate with ultraviolet rays in which an exposure amount at 365 nm is 1,000 mJ/cm$^2$, and then heating the obtained cured product in a nitrogen gas atmosphere at 80°C for 30 minutes.

<Method for producing curable composition>

The curable composition according to the present invention can be obtained by mixing the polyfunctional (meth) acrylate thioester composition with the compound (B) and other components as necessary by a known method in the related art.

From the viewpoint of further improving the performance balance of high refractive index, low curing shrinkage, and glass adhesiveness of a cured product to be obtained, in a case where the total of the curable composition is set to 100% by mass, the total content of the polyfunctional (meth)acrylate thioester composition and the compound (B) in the curable composition according to the present invention is preferably 70% by mass or more, more preferably 80% by mass or more, still more preferably 90% by mass or more, and even still more preferably 95% by mass or more, and is preferably 100% by mass or less.

[Cured product]

The cured product according to the present invention is obtained by curing the curable composition according to the present invention. Since the cured product according to the present invention has improved performance balance of high refractive index, low curing shrinkage, and glass adhesiveness, the cured product can be suitably used as an optical material.

From the viewpoint of further improving transparency of the cured product, in a case where a test piece having a thickness of 250 μm is produced from the cured product according to the present embodiment, a light transmittance ($T_1$) of the test piece at a wavelength of 400 nm is preferably 81% or more, more preferably 83% or more, still more preferably 84% or more, and even more preferably 85% or more. The upper limit value of the light transmittance ($T_1$) is not particularly limited, but is, for example, less than 100%, and may be 99% or less, 95% or less, or 90% or less.

In the present embodiment, the test piece having a thickness of 250 μm, which is formed of the cured product according to the present embodiment, can be obtained, for example, by irradiating a curable film formed of the curable composition applied onto a glass substrate with ultraviolet rays in which an exposure amount at 365 nm is 1,000 mJ/cm$^2$, and then heating the obtained cured product in a nitrogen gas atmosphere at 80°C for 30 minutes.

In addition, in a case where a light transmittance of the test piece having a thickness of 250 μm, which is produced from the cured product according to the present embodiment and then heated in an oven set to 125°C in an air atmosphere for 168 hours, at a wavelength of 400 nm is denoted as $T_2$, a rate of change in light transmittance after the heating, which is represented by $(T_1 - T_2)/T_1 \times 100$, is preferably 7.0% or less, more preferably 5.0% or less, still more preferably 4.0% or less, and even more preferably 3.0% or less. The lower limit value of the rate of change in light transmittance after the heating is not particularly limited, but is, for example, 0.1% or more, and may be 0.5% or more, 0.8% or more, or 1.0% or more.

From the viewpoint of further improving optical characteristics of the cured product, in a case where the test piece having a thickness of 250 μm is produced from the cured product according to the present embodiment, a refractive index (nD) of the test piece with respect to D-line (589.3 nm) is preferably 1.600 or more, more preferably 1.610 or more, still more preferably 1.620 or more, even more preferably 1.630 or more, even still more preferably 1.635 or more, further more preferably 1.640 or more, and even further more preferably 1.645 or more. The upper limit value of the refractive index (nD) of the test piece with respect to the D-line (589.3 nm) is not particularly limited, but is, for example, 1.700 or less, and may be 1.680 or less, 1.655 or less, or 1.650 or less.

From the viewpoint of further improving optical characteristics of the cured product, in a case where the test piece having a thickness of 250 μm is produced from the cured product according to the present embodiment, an Abbe number (vD) of the test piece, which is measured in accordance with ASTM D542, is preferably 20 or more, more preferably 25 or more, still more preferably 28 or more, even more preferably 30 or more, even still more preferably 32 or more, and further more preferably 33 or more. The upper limit value of the Abbe number (vD) of the test piece is not particularly limited, but is, for example, 45 or less, and may be 42 or less, 40 or less, or 38 or less.

[Molded body]

The molded body according to the present invention is a molded body including the cured product according to the present invention, and can be obtained, for example, by molding the curable composition according to the present invention into a predetermined shape while curing the curable composition.

Since the molded body according to the present invention includes the cured product according to the present

invention, the performance balance of high refractive index, low curing shrinkage, and glass adhesiveness is improved, and thus the molded body can be suitably used as an optical material.

From the viewpoint of further improving the performance balance of high refractive index, low curing shrinkage, and glass adhesiveness, in a case where the total of the molded body is set to 100% by mass, a content of the cured product according to the present invention in the molded body according to the present invention is preferably 50% by mass or more, more preferably 70% by mass or more, still more preferably 80% by mass or more, even more preferably 90% by mass or more, even still more preferably 95% by mass or more, and further more preferably 97% by mass or more, and is preferably 100% by mass or less.

[Optical material]

The optical material according to the present invention is an optical material including the molded body according to the present invention, and can be obtained, for example, by molding the curable composition according to the present invention into a predetermined shape while curing the curable composition.

Since the optical material according to the present invention includes the cured product or molded body according to the present invention, the performance balance of high refractive index, low curing shrinkage, and glass adhesiveness is improved. Therefore, the optical material according to the present invention can be used for various optical lenses such as lenses for various sensors, pickup lenses, projector lenses, prisms, fθ lenses, imaging lenses, camera lenses, light guide plates, head-mounted display lenses, plastic eyeglass lenses, goggles, vision correction eyeglass lenses, lenses for imaging equipment, Fresnel lenses for liquid crystal projectors, lenticular lenses, and contact lenses; sealing materials for light emitting diodes (LEDs); optical adhesives used for optical waveguides, wafer-level optical components (WLOs), and optical waveguide bonding; antireflection films used for optical lenses and the like; transparent coatings used for liquid crystal display device members (substrates, light guide plates, films, sheets, and the like); sheets or films attached to windshields of cars or helmets of motorcycles; and transparent substrates.

Since the optical material according to the present invention has improved the performance balance of high refractive index, low curing shrinkage, and glass adhesiveness, the optical material can be more suitably used as various optical lenses.

Furthermore, since the optical material according to the present invention has improved the performance balance of high refractive index, low curing shrinkage, and glass adhesiveness, the optical material according to the present invention can be suitably used as a laminate of the molded body according to the present invention and a glass substrate, and can be suitably used as a so-called hybrid-type optical lens in which a resin lens is formed on a glass substrate.

The embodiments of the present invention have been described above, but these are examples of the present invention and various configurations other than the above can be adopted.

In addition, the present invention is not limited to the above-described embodiments, and modifications, improvements, and the like within the range in which the object of the present invention can be achieved are included in the present invention. Examples

Hereinafter, the present embodiment will be described in detail with reference to Examples and the like. It should be noted that the present embodiment is not limited to the descriptions of these examples.

<Example 1>

(Preparation of curable composition)

A composition obtained in Synthesis Example 2 (100 parts by weight) as a polyfunctional (meth)acrylate thioester composition, and Irg184 (1-hydroxycyclohexyl phenyl ketone, manufactured by BASF, 3.0 parts by weight) as a polymerization initiator were added to a sample bottle, and mixed using a mixing rotor until the appearance was uniform, thereby obtaining a curable composition.

Using an E-type viscometer (TVE-25L, manufactured by Toki Sangyo Co., Ltd.), the viscosity of the obtained curable composition at 25°C was measured under a condition of a rotation speed of 2.5 rpm.

(Production of cured film)

0.5 mL of the curable composition was applied onto an Eagle-XG (alkali-free glass substrate manufactured by CORNING, 70 × 70 × 0.7 mmT) which had been subjected to a mold release treatment using Novec 1720 (fluorosilane-based coating agent manufactured by 3M Company), the curable composition was sandwiched between the glass substrate and another glass substrate which had been subjected to a mold release treatment through a spacer having a thickness of 250 μm, and an end part was fixed with a clip.

The obtained laminate was irradiated with ultraviolet rays from one surface thereof using a non-electrode lamp (H bulb) such that an exposure amount at 365 nm was 1,000 $mJ/cm^2$, and the cured product was released from the glass substrate and heated in a nitrogen gas atmosphere at 80°C for 30 minutes to obtain a cured film having a thickness of 250 μm.

A refractive index of the obtained cured film and a shrinkage rate during curing were measured by the following methods. The obtained results are shown in Table 1.

<Measurement of refractive index (nD) and Abbe number (vD)>

The refractive index (nD) and the Abbe number (vD) were measured in accordance with ASTM D542 as follows.

The refractive index (nD) of the cured film with respect to the D-line (589.3 nm) was measured using an Abbe refractometer (DR-M2, manufactured by ATAGO CO., LTD.). RE-3520 (D-line, manufactured by ATAGO CO., LTD.) was used as an interference filter and RE-1196 (monobromonaphthalene, manufactured by ATAGO CO., LTD.) was used as an intermediate liquid, and the measurement was performed by setting the sample temperature to 25°C.

In addition, a refractive index (nC) of the cured film with respect to C-line (wavelength: 656.3 nm) was measured using an Abbe refractometer (manufactured by ATAGO CO., LTD., DR-M2) with RE-3522 (C-line, manufactured by ATAGO CO., LTD.) as an interference filter and RE-1196 (monobromonaphthalene, manufactured by ATAGO CO., LTD.) as an intermediate liquid, with a sample temperature set to 25°C.

In addition, a refractive index (nF) of the cured film with respect to C-line (wavelength: 486.1 nm) was measured using a refractometer (manufactured by ATAGO CO., LTD., DR-M2) with RE-3521 (F-line, manufactured by ATAGO CO., LTD.) as an interference filter and RE-1196 (monobromonaphthalene, manufactured by ATAGO CO., LTD.) as an intermediate liquid, with a sample temperature set to 25°C.

Thereafter, the Abbe number of the cured film was calculated according to the following expression.

$$\text{Abbe number} = (nD - 1)/(nF - nC)$$

<Light transmittance>

A light transmittance of the obtained cured film in a thickness direction was measured under the following conditions to obtain a light transmittance $T_1$. The measurement was performed by attaching the cured film to an integrating sphere, and an incidence surface was set to an arbitrary surface.

(Measurement condition)

Measuring device: UH4150 (ultraviolet-visible-near infrared spectrophotometer manufactured by Hitachi High-Tech Science Corporation)
Measurement method: transmission method
Measurement wavelength: 400 nm
Reference: air
Detector: integrating sphere/photomultiplier (200 nm to 850 nm)
Integrating sphere: PbS (850 nm to 2600 nm)

<Rate of change in light transmittance after heating (125°C and 168 hours)>

First, the obtained cured film was heated in an oven set to 125°C in an air atmosphere for 168 hours. Thereafter, a light transmittance $T_2$ after the heating was obtained by measuring the light transmittance in the thickness direction under the above-described conditions, and the rate of change in light transmittance after the heating was calculated by the following expression. The results are shown in Table 1.

Rate of change in light transmittance after heating (125°C and 168 hours) (%) = $(T_1 - T_2)/T_1 \times 100$

<Measurement of curing shrinkage rate>

A specific gravity $d^1$ of the curable composition was measured using a pycnometer (JIS Z 8804:2012). In addition, a specific gravity $d^2$ of the cured film was measured by an Archimedes method (JIS Z 8807:2012). Using the values of the

specific gravities, a curing shrinkage rate (%) was calculated by the following expression.

$$\text{Expression: curing shrinkage rate (\%)} = (1 - d^1/d^2) \times 100$$

<Evaluation of glass adhesiveness>

0.5 mL of the curable composition was applied onto an Eagle-XG (alkali-free glass substrate manufactured by CORNING, 70 × 70 × 0.7 mmT) which had been subjected to a mold release treatment using Novec 1720 (fluorosilane-based coating agent manufactured by 3M Company), the curable composition was sandwiched between the glass substrate and an EAGLE-XG (alkali-free glass substrate manufactured by CORNING, 70 × 70 × 0.7 mmT) which had not been subjected to a mold release treatment through a spacer having a thickness of 250 μm, and an end part was fixed with a clip.

The obtained laminate was irradiated with ultraviolet rays from one surface thereof using a non-electrode lamp (H bulb) such that an exposure amount at 365 nm was 1,000 mJ/$cm^2$, and the cured product was released from the mold release-treated glass substrate and heated in a nitrogen gas atmosphere at 80°C for 30 minutes to obtain a laminate of resin cured product/glass substrate.

The obtained laminate was evaluated for glass adhesiveness based on the following evaluation standard. The evaluation results are shown in Table 1.

(Evaluation standard)

PASS (good): peeling at an interface between the resin and the glass substrate or cracks in the resin were not observed.

FAIL (poor): peeling at an interface between the resin and the glass substrate or cracks in the resin were observed.

<Examples 2 to 5 and Comparative Examples 1 and 2>

Each evaluation was performed in the same manner as in Example 1, except that the composition of the curable composition was changed to the composition shown in Table 1. Table 1 shows the composition of the curable composition and various evaluation results.

[Table 1]

Table 1

| | Component name | Example 1 | Example 2 | Example 3 | Example 4 | Example 5 | Comparative Example 1 | Comparative Example 2 |
|---|---|---|---|---|---|---|---|---|
| Composition of curable composition (part by mass) | Composition synthesized in Synthesis Example 2 | 100 | | | | 100 | | |
| | Composition synthesized in Synthesis Example 3 | | 100 | 80 | 60 | | | |
| | Compound (B) A-BPEF | | | 20 | 40 | | | |
| | GSTA | | | | | | 100 | |
| | Composition synthesized in Synthesis Example 4 | | | | | | | 100 |
| | Irg184 | 3 | 3 | 3 | 3 | 3 | 3 | 3 |
| | KBM-5103 | | | | | 1 | | |
| | A0-60 | | | | | 0.5 | | |
| | LA-82 | | | | | 0.1 | | |
| | LA-46 | | | | | 0.05 | | |
| Viscosity of curable composition (mPa·s, 25°C) | | 6200 | 1600 | 2800 | 6000 | 6000 | 370 | 2100 |
| Physical properties of cured product | Refractive index (nD, 25°C) | 1.649 | 1.649 | 1.642 | 1.637 | 1.640 | 1.642 | 1.651 |
| | Abbe number | 36 | 34 | 32 | 30 | 36 | 35 | 31 |
| | Curing shrinkage rate (%) | 6.8 | 6.1 | 6.3 | 6.2 | 6.2 | 9.6 | 11.2 |
| | Glass adhesiveness | Pass | Pass | Pass | Pass | Pass | Fail | Fail |
| | Light transmittance (%) | 84.3 | 84.1 | 86.5 | 86.7 | 88.7 | 80.1 | 79.1 |
| | Rate of change in light transmittance after heating (125°C and 168 hours) (%) | 3.5 | 3.6 | 2.1 | 1.2 | 1.1 | 8 | 9 |

Details of each component shown in Table 1 are as follows.

· GSTA: 1,8-bisacryloylthio-(4-acryloylthiomethyl-3,6-dithiaoctane (produced according to Synthesis Example 1 described later)

· A-BPEF (fluorene-based acrylate monomer (9,9-bis(4-(2-acryloyloxyethoxy)phenyl)fluorene), manufactured by Shin-Nakamura Chemical Co., Ltd.; compound having the following structure)

[Chem. 15]

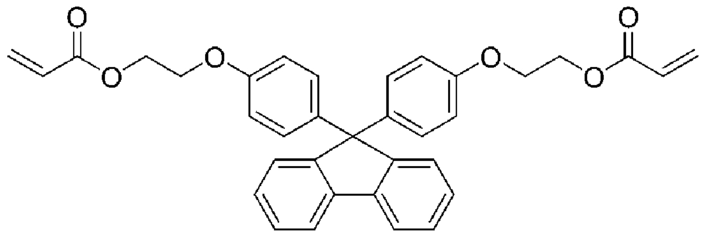

(Other components)

· Irg184: polymerization initiator (1-hydroxycyclohexyl phenyl ketone, manufactured by BASF; compound having the following structure)

[Chem. 16]

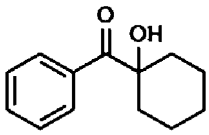

· KBM-5103: silane coupling agent (3-acryloxypropyltrimethoxysilane, manufactured by Shin-Etsu Chemical Co., Ltd.; compound having the following structure)

[Chem. 17]

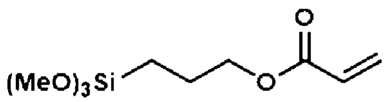

· AO-60: antioxidant (pentaerythritol-tetrakis[3-(3,5-di-tert-butyl-4-hydroxyphenyl)propionate], manufactured by ADEKA Corporation; compound having the following structure)

[Chem. 18]

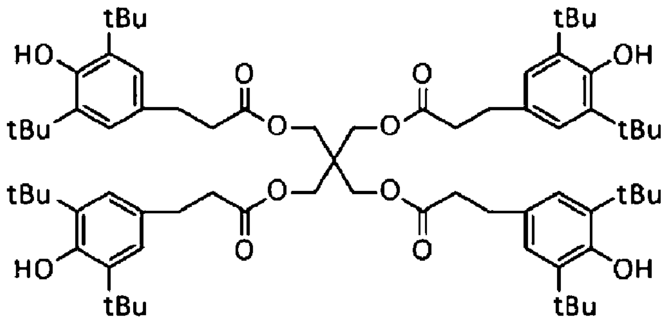

· LA-82: hindered amine-based light stabilizer ((1,2,2,6,6-pentamethyl-piperidin-4-yl) methacrylic acid, manufactured by ADEKA Corporation, compound having the following structure)

[Chem. 19]

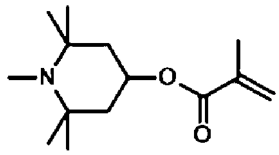

· LA-46: ultraviolet absorber (2-(4,6-diphenyl-1,3,5-triazin-2-yl)-5-[2-(2-ethylhexanoyloxy)ethoxy]phenol, compound having the following structure, manufactured by ADEKA Corporation)

[Chem. 20]

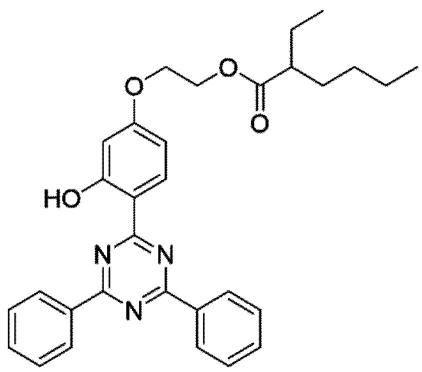

(Synthesis Example 1: synthesis of GSTA)

4-Mercaptomethyl-1,8-dimercapto-3,6-dithiaoctane (GST, 100.0 g, 383.9 mmol) was charged into a four-neck flask equipped with a stirrer, a thermometer, a nitrogen introduction line, and a dropping funnel, and diluted with dichloromethane (100 mL), and stirring the mixture was started. Next, 3-chloropropionyl chloride (121.84 g, 959.7 mmol) was added dropwise thereto while cooling the reaction solution with an ice bath so that the internal temperature was 40°C or lower.

The reaction solution was stirred at room temperature for 48 hours, pure water (100 mL) was added thereto, and the organic phase was separated by a liquid separation operation. The obtained organic phase was washed twice with a sodium hydrogen carbonate-saturated aqueous solution (100 mL), and the solvent was distilled off by an evaporator to obtain a compound (201.0 g) having the following structure.

[Chem. 21]

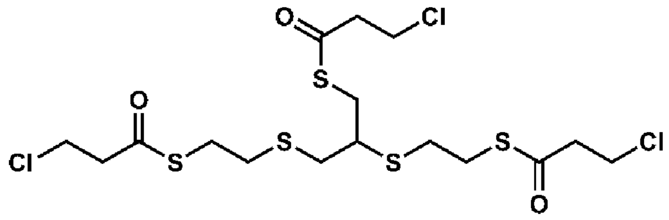

The obtained compound was charged into a four-neck flask equipped with a thermometer and a dropping funnel, 4-methoxyphenol (150 mg) as a polymerization inhibitor was added thereto, and the mixture was dissolved while stirring at room temperature. Next, triethylamine (116.5 g, 1152 mmol) was added dropwise thereto while cooling the reaction solution with an ice bath so that the internal temperature was 40°C or lower.

The reaction solution was stirred at room temperature for 1 hour, 1 M hydrochloric acid (300 mL) was added thereto, and the organic phase was separated by a liquid separation operation. The obtained organic phase was allowed to pass through silica gel (100 mL), 4-methoxyphenol (150 mg) as a polymerization inhibitor was added thereto, and the resulting mixture was concentrated under reduced pressure to obtain a colorless and transparent compound (GSTA) (110.0 g) having the following structure. In a case where a molecular weight thereof was measured by gel permeation chromatography (GPC), a number-average molecular weight (Mn) was 400 and a weight-average molecular weight (Mw) was 440.

[Chem. 22]

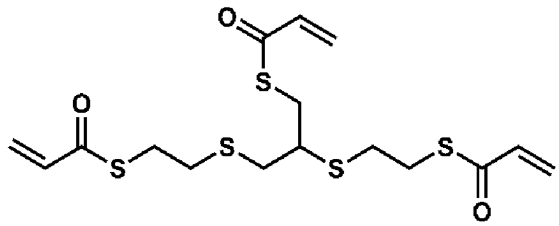

(Synthesis Example 2)

GSTA (9.0 g) obtained in Synthesis Example 1 and bis(2-mercaptoethyl)sulfide (1.0 g) were charged into a four-neck flask equipped with a stirrer, a thermometer, a nitrogen introduction line, and a dropping funnel. Next, triethylamine (10 mg) was added thereto while cooling the reaction solution with an ice bath so that the internal temperature was 30°C or lower. The reaction solution was stirred at room temperature for 24 hours, 1 M hydrochloric acid (100 mL) and dichloromethane (100 mL) were added thereto, and the organic phase was separated by a liquid separation operation. The obtained organic phase was allowed to pass through silica gel (10 mL), 4-methoxyphenol (10 mg) as a polymerization inhibitor was added thereto, and the resulting mixture was concentrated under reduced pressure to obtain a colorless and transparent polyfunctional (meth)acrylate thioester composition (9.9 g) containing the following compound. In a case where a molecular weight thereof was measured by GPC, a number-average molecular weight (Mn) was 600 and a weight-average molecular weight (Mw) was 3,500.

[Chem. 23]

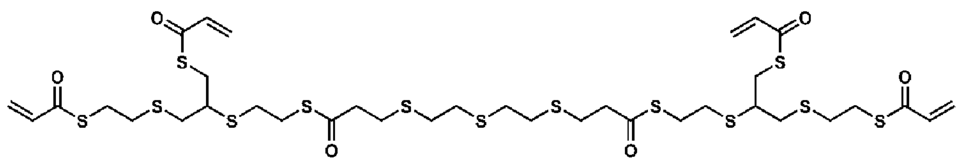

(Synthesis Example 3)

GST (trithiol, 100.0 g, 383.9 mmol) was charged into a four-neck flask equipped with a stirrer, a thermometer, a nitrogen introduction line, and a dropping funnel, and diluted with dichloromethane (100 mL), and stirring the mixture was started. Next, 3-chloropropionyl chloride (121.84 g, 959.7 mmol) was added dropwise thereto while cooling the reaction

solution with an ice bath so that the internal temperature was 40°C or lower.

The reaction solution was stirred at room temperature for 48 hours, pure water (100 mL) was added thereto, and the organic phase was separated by a liquid separation operation. The obtained organic phase was washed twice with a sodium hydrogen carbonate-saturated aqueous solution (100 mL) to obtain a dichloromethane solution of a composition containing GST (compound group shown below) in which some of thiol groups of GST were esterified with 3-chloropropionic acid. A peak area ratio of 3-substituted isomer:2-substituted isomer:1-substituted isomer in the high-performance liquid chromatography (HPLC) measurement was 56:38:6.

[Chem. 24]

3-substituted isomer

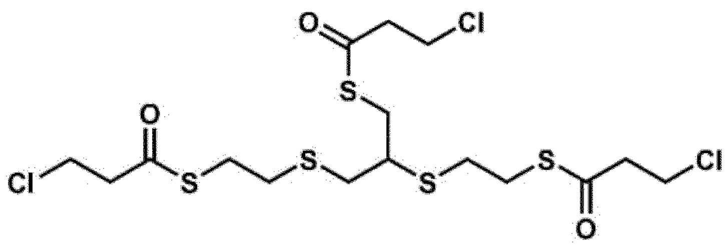

[Chem. 25]

2-substituted isomer

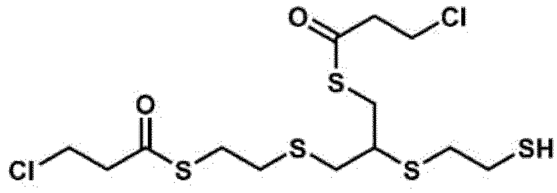

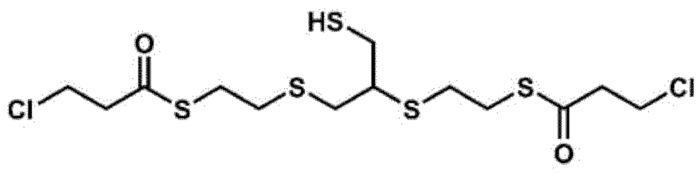

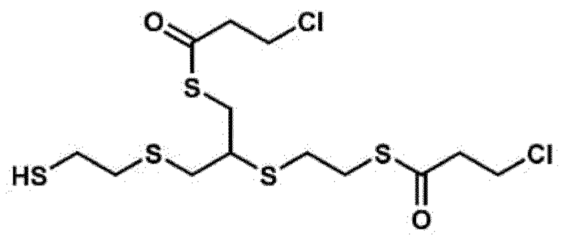

[Chem. 26]

1-substituted isomer

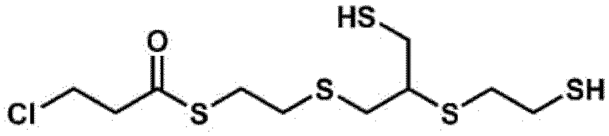

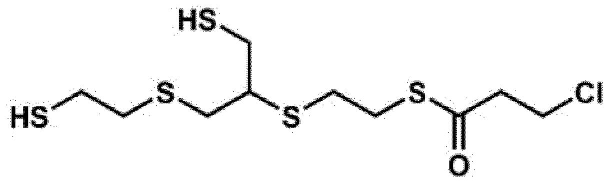

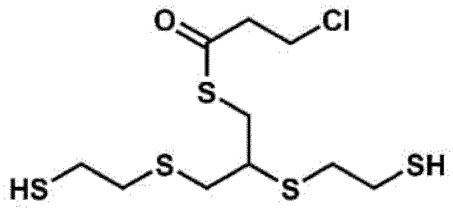

The obtained solution was charged into a four-neck flask equipped with a stirrer, a thermometer, and a dropping funnel, 4-methoxyphenol (150 mg) as a polymerization inhibitor was added thereto, and the mixture was dissolved while stirring at room temperature. Next, triethylamine (116.5 g, 1152 mmol) was added dropwise thereto while cooling the reaction solution with an ice bath so that the internal temperature was 40°C or lower.

The reaction solution was stirred at room temperature for 1 hour, 1 M hydrochloric acid (300 mL) was added thereto, and the organic phase was separated by a liquid separation operation. The obtained organic phase was allowed to pass through silica gel (100 mL), 4-methoxyphenol (150 mg) as a polymerization inhibitor was added thereto, and the resulting mixture was concentrated under reduced pressure to obtain a colorless and transparent polyfunctional (meth)acrylate thioester composition (132.5 g) containing the following compound. In a case where a molecular weight thereof was measured by GPC, a number-average molecular weight (Mn) was 500 and a weight-average molecular weight (Mw) was 1,600.

[Chem. 27]

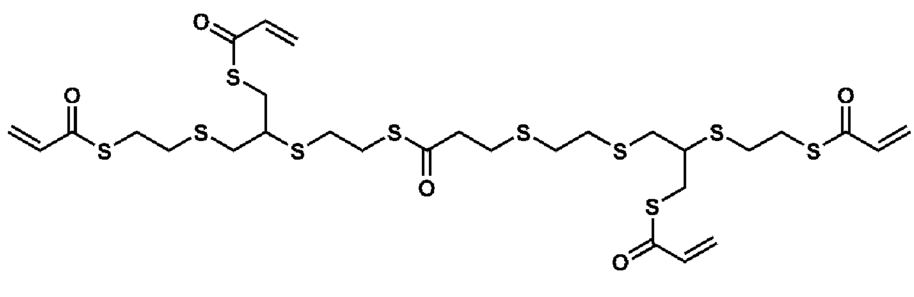

(Synthesis Example 4)

MES (bis(2-mercaptoethyl)sulfide, 100.0 g, 648.1 mmol) was charged into a four-neck flask equipped with a stirrer, a thermometer, a nitrogen introduction line, and a dropping funnel, and diluted with dichloromethane (100 mL), and stirring the mixture was started. Next, 3-chloropropionyl chloride (123.42 g, 972.1 mmol) was added dropwise thereto while cooling the reaction solution with an ice bath so that the internal temperature was 40°C or lower.

The reaction solution was stirred at room temperature for 48 hours, pure water (100 mL) was added thereto, and

the organic phase was separated by a liquid separation operation. The obtained organic phase was washed twice with a sodium hydrogen carbonate-saturated aqueous solution (100 mL) to obtain a dichloromethane solution of a composition containing MES (compound group shown below) in which some of thiol groups were esterified with 3-chloropropionic acid.

[Chem. 28]

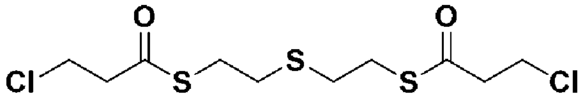

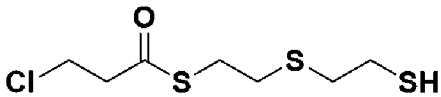

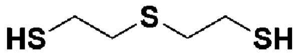

The obtained solution was charged into a four-neck flask equipped with a stirrer, a thermometer, and a dropping funnel, 4-methoxyphenol (150 mg) as a polymerization inhibitor was added thereto, and the mixture was dissolved while stirring at room temperature. Next, triethylamine (144.28 g, 1430 mmol) was added dropwise thereto while cooling the reaction solution with an ice bath so that the internal temperature was 40°C or lower.

The reaction solution was stirred at room temperature for 1 hour, 1 M hydrochloric acid (300 mL) was added thereto, and the organic phase was separated by a liquid separation operation. The obtained organic phase was allowed to pass through silica gel (100 mL), 4-methoxyphenol (150 mg) as a polymerization inhibitor was added thereto, and the resulting mixture was concentrated under reduced pressure to obtain a colorless and transparent polyfunctional (meth) acrylate thioester composition (121.5 g) containing the following compound. In a case where a molecular weight thereof was measured by GPC, a number-average molecular weight (Mn) was 700 and a weight-average molecular weight (Mw) was 2,000.

[Chem. 29]

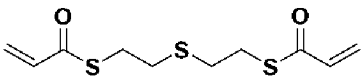

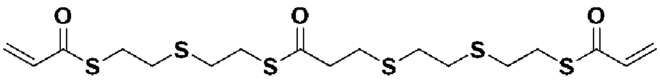

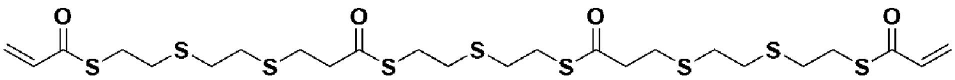

(Molecular weight measurement)

The number-average molecular weight (Mn) and the weight-average molecular weight (Mw) of the polyfunctional (meth)acrylate thioester compositions in Synthesis Examples 1 to 4 were measured by gel permeation chromatography (GPC) according to the following procedure.

(1) Preparation of sample solution

The polyfunctional (meth)acrylate thioester composition was dissolved in tetrahydrofuran such that the concentration was 1 g/100 mL. Next, the solution was filtered using a filter (manufactured by Membrane Solutions, product name: syringe filter PTFE 013100) having a pore size of 1 μm to remove insoluble components, thereby obtaining a sample solution.

(2) Measurement of molecular weight

Using a GPC measuring device (product name: Alliance, manufactured by WATERS Corporation), tetrahydrofuran (manufactured by FUJIFILM WAKO Pure Chemical Corporation, for high performance liquid chromatography) as a mobile phase was allowed to flow at a flow rate of 1.0 mL per minute, and three-connected analysis columns (gel permeation columns, manufactured by Agilent Technologies, Inc., product name: PLgel 5 μm Mixed-C) were stabilized in a constant temperature bath at 40°C. 10 μL of the sample solution was injected into the column to perform the measurement. A differential refractive index (RI) detector was used as a detector. The molecular weight of the sample was calculated based on a calibration curve prepared in advance. As the calibration curve at this time, a calibration curve was used in which a plurality of kinds of monodisperse polystyrene (manufactured by Agilent Technologies, Inc.) having a known molecular weight were used as a standard sample.

Priority is claimed on Japanese Patent Application No. 2022-052197, filed March 28, 2022, the disclosure of which is incorporated herein by reference.

## Claims

1. A polyfunctional (meth)acrylate thioester composition comprising:

   a polyfunctional (meth)acrylate thioester compound (A) represented by Formula (1),

   [Chem. 1]

   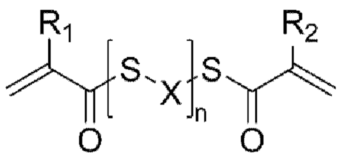

   (1)

   (in Formula (1), n represents an integer of 5 or more, X represents an alkylene group having 1 or more and 4 or less carbon atoms, in which any methylene group may be substituted with a carbonyl group, provided that any hydrogen atom in the alkylene group is substituted with a structure of Formula (2), and $R_1$ and $R_2$ each independently represent a hydrogen atom or a methyl group),

   [Chem. 2]

   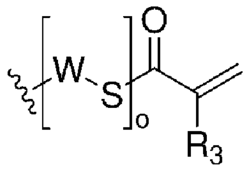

   (2)

   (in Formula (2), $R_3$ represents a hydrogen atom or a methyl group, a plurality of $R_3$'s may be the same or different from each other, W represents an alkylene group having 1 or more and 4 or less carbon atoms, and o represents an integer of 1 or more).

2. The polyfunctional (meth)acrylate thioester composition according to Claim 1, wherein a weight-average molecular weight of the polyfunctional (meth)acrylate thioester composition is 1,000 or more and 100,000 or less.

3. The polyfunctional (meth)acrylate thioester composition according to Claim 1 or 2,

   wherein the polyfunctional (meth)acrylate thioester compound (A) includes at least one selected from the group consisting of a compound represented by Formula (3) and a compound represented by Formula (4),

[Chem. 3]

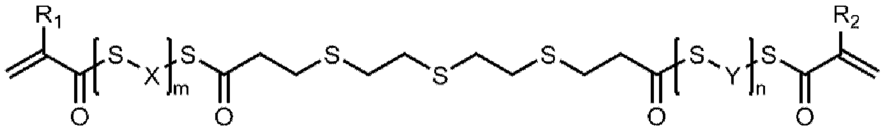

(3)

[Chem. 4]

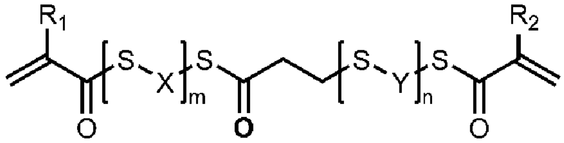

(4)

(in Formulae (3) and (4), m and n each independently represent an integer of 2 or more, X and Y each independently represent an alkylene group having 1 or more and 4 or less carbon atoms, in which any hydrogen atom may be substituted with the structure of Formula (2), a plurality of X's and a plurality of Y's may be each the same or different from each other, and $R_1$ and $R_2$ each independently represent a hydrogen atom or a methyl group).

**4.** A curable composition comprising:
the polyfunctional (meth)acrylate thioester composition according to any one of Claims 1 to 3.

**5.** The curable composition according to Claim 4, further comprising:

a compound (B) represented by Formula (5),

[Chem. 5]

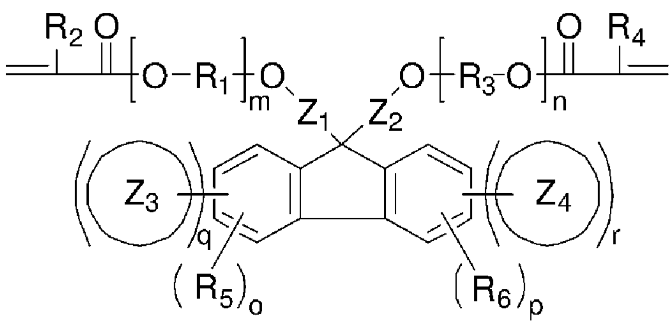

(5)

(in Formula (5), $Z_1$ and $Z_2$ each independently represent an aromatic carbon ring or an alkylene group, provided that any hydrogen atom may be substituted with an alkyl group or an alkoxy group, $Z_3$ and $Z_4$ each independently represent an aromatic carbon ring or an alkyl group, provided that any hydrogen atom may be substituted with an alkyl group or an alkoxy group, $R_1$ and $R_3$ each independently represent an alkylene group having 1 or more and 10 or less carbon atoms, $R_2$ and $R_4$ each independently represent a hydrogen atom or a methyl group, $R_5$ and $R_6$ each independently represent an alkyl group, m and n each independently represent an integer of 0 or more, and o, p, q, and r each represent an integer of 0 or more, satisfying relationships of $o + q \leq 4$ and $p + r \leq 4$).

**6.** The curable composition according to Claim 4 or 5,
wherein a viscosity of the curable composition at 25°C, which is measured using an E-type viscometer under a condition of a rotation speed of 2.5 rpm, is 100 mPa·s or more and 10,000 mPa·s or less.

**7.** The curable composition according to any one of Claims 4 to 6, further comprising:
a polymerization initiator.

**8.** The curable composition according to any one of Claims 4 to 7, further comprising: at least one selected from the group consisting of a silane coupling agent, an antioxidant, an ultraviolet absorber, and a light stabilizer.

**9.** The curable composition according to any one of Claims 4 to 8, wherein, in a case where a specific gravity of the curable composition, which is measured using a pycnometer in accordance with JIS Z 8804:2012, is denoted as $d^1$, and a specific gravity of a test piece having a thickness of 250 μm, which is formed of a cured product of the curable composition, is denoted as $d^2$, a curing shrinkage rate of the curable composition, which is represented by $(1 - d^1/d^2) \times 100$, is 9.0% or less.

**10.** The curable composition according to any one of Claims 4 to 9, wherein the curable composition is usable in an optical material.

**11.** A cured product obtained by curing the curable composition according to any one of Claims 4 to 10.

**12.** The cured product according to Claim 11, wherein, in a case where a test piece having a thickness of 250 μm is produced from the cured product, a light transmittance ($T_1$) of the test piece at a wavelength of 400 nm is 81% or more.

**13.** The cured product according to Claim 12, wherein, in a case where a light transmittance at a wavelength of 400 nm of the test piece after being heated in an oven set to 125°C in an air atmosphere for 168 hours is denoted as $T_2$, a rate of change in light transmittance after the heating, which is represented by $(T_1 - T_2)/T_1 \times 100$, is 7.0% or less.

**14.** The cured product according to any one of Claims 11 to 13, wherein, in a case where a test piece having a thickness of 250 μm is produced from the cured product, a refractive index (nD) of the test piece with respect to D-line (589.3 nm) is 1.600 or more.

**15.** The cured product according to any one of Claims 11 to 14, wherein, in a case where a test piece having a thickness of 250 μm is produced from the cured product, an Abbe number (νD) of the test piece, which is measured in accordance with ASTM D542, is 20 or more.

**16.** A molded body comprising: the cured product according to any one of Claims 11 to 15.

**17.** An optical material comprising: the molded body according to Claim 16.

**18.** The optical material according to Claim 17, further comprising:

a glass substrate,
wherein the optical material is a laminate of the molded body and the glass substrate.

**19.** The optical material according to Claim 17 or 18, wherein the optical material is an optical lens.

**20.** A method for producing the polyfunctional (meth)acrylate thioester composition according to any one of Claims 1 to 3, the method comprising:

a step of converting, among total thiol groups in a polythiol compound, 0.5 equivalent or more and 0.9 equivalent or less of thiol groups into a functional group represented by the following structural formula and subjecting the obtained compound to a β-elimination reaction and an ene-thiol reaction to synthesize a polyfunctional (meth)acrylate thioester compound,

[Chem. 6]

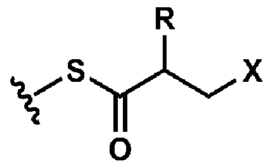

(in the structural formula, X represents a fluorine atom, a chlorine atom, a bromine atom, or an iodine atom, and R represents a hydrogen atom or a methyl group).

**21.** A method for producing the polyfunctional (meth)acrylate thioester composition according to any one of Claims 1 to 3, the method comprising:
a step of reacting a polythiol compound with a polythio(meth)acrylate compound by an ene-thiol reaction.

**INTERNATIONAL SEARCH REPORT**

International application No. **PCT/JP2023/005701**

**A. CLASSIFICATION OF SUBJECT MATTER**

***C08F 20/38***(2006.01)i; ***B32B 17/10***(2006.01)i; ***B32B 27/30***(2006.01)i; ***C07C 327/22***(2006.01)i; ***C08F 220/20***(2006.01)i; ***C08G 75/02***(2016.01)i; ***G02B 1/04***(2006.01)i
FI: C08F20/38; B32B17/10; B32B27/30 A; C07C327/22; C08F220/20; C08G75/02; G02B1/04

According to International Patent Classification (IPC) or to both national classification and IPC

**B. FIELDS SEARCHED**

Minimum documentation searched (classification system followed by classification symbols)

C08F20/38; B32B17/10; B32B27/30; C07C327/22; C08F220/20; C08G75/02; G02B1/04

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | US 6172140 B1 (SOLA INTERNATIONAL HOLDINGS LTD.) 09 January 2001 (2001-01-09)<br>entire text | 1-21 |
| A | JP 10-204056 A (MITSUI CHEMICALS, INC.) 04 August 1998 (1998-08-04)<br>entire text | 1-21 |
| A | JP 2004-176006 A (SUMITOMO SEIKA CHEMICALS CO., LTD.) 24 June 2004 (2004-06-24)<br>entire text | 1-21 |
| A | JP 2011-162610 A (OSAKA GAS CHEMICALS CO., LTD.) 25 August 2011 (2011-08-25)<br>entire text | 1-21 |
| A | JP 2004-2820 A (MITSUI CHEMICALS, INC.) 08 January 2004 (2004-01-08)<br>entire text | 1-21 |
| A | JP 7-252207 A (MITSUI TOATSU CHEM., INC.) 03 October 1995 (1995-10-03)<br>entire text | 1-21 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 April 2023** | **16 May 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

## INTERNATIONAL SEARCH REPORT
**Information on patent family members**

International application No.
**PCT/JP2023/005701**

| Patent document cited in search report | Publication date (day/month/year) | Patent family member(s) | Publication date (day/month/year) |
|---|---|---|---|
| US 6172140 B1 | 09 January 2001 | WO 1998/024761 A1 | |
| JP 10-204056 A | 04 August 1998 | (Family: none) | |
| JP 2004-176006 A | 24 June 2004 | (Family: none) | |
| JP 2011-162610 A | 25 August 2011 | (Family: none) | |
| JP 2004-2820 A | 08 January 2004 | US 2005/0131203 A1<br>entire text<br>EP 1505094 A1<br>CN 1578798 A | |
| JP 7-252207 A | 03 October 1995 | US 5608115 A<br>entire text<br>EP 665219 A1<br>CN 1111619 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 199824761 A
- JP H08325337 A
- JP 2022052197 A